# EUROPEAN PATENT SPECIFICATION

(11) **EP 3 106 113 B1**
(45) Date of publication and mention of the grant of the patent: **10.11.2021**
(21) Application number: 16163749.1
(22) Date of filing: 28.02.2013
(51) Int. Cl.: A61B 17/285, A61B 17/32, A61B 18/14, A61B 17/3205, A61B 18/00

(54) **VESSEL SEALING INSTRUMENT**
GEFÄSSVERSIEGELUNGSVORRICHTUNG
INSTRUMENT D'OBTURATION DES VAISSEAUX

(30) Priority: 08.03.2012 US 201213415471
(43) Date of publication of application: 21.12.2016
(62) Divisional of application: 13757413.3
(73) Proprietor: Covidien LP, Mansfield, MA 02048 (US)
(72) Inventor: SHARP, Robert M., Boulder, Colorado 80304 (US); ARTALE, Ryan C., Boulder, Colorado 80305 (US); MOUA, Tony, Broomfield, Colorado 80020 (US)
(74) Representative: Morgan, Marc

(56) References cited:
- US-A1- 2005 107 784
- US-A1- 2008 215 048
- US-A1- 2009 182 327
- US-A1- 2009 240 246
- None

## Description

### BACKGROUND

### 1. Background of Related Art

The present disclosure relates to forceps used for open surgical procedures. More particularly, the present disclosure relates to a forceps that applies electrosurgical current to seal tissue.

### 2. Technical Field

A hemostat or forceps is a simple plier-like tool which uses mechanical action between its jaws to constrict vessels and is commonly used in open surgical procedures to grasp, dissect and/or clamp tissue. Electrosurgical forceps utilize both mechanical clamping action and electrical energy to effect hemostasis by heating the tissue and blood vessels to coagulate, cauterize and/or seal tissue.

Certain surgical procedures require sealing and cutting blood vessels or vascular tissue, Several journal articles have disclosed methods for sealing small blood vessels using electrosurgery. An article entitled Studies on Coagulation and the Development of an Automatic Computerized Bipolar Coagulator, J. Neurosurg., Volume 75, July 1991, describes a bipolar coagulator which is used to seal small blood vessels. The article states that it is not possible to safely coagulate arteries with a diameter larger than 2 to 2.5 mm. A second article is entitled Automatically Controlled Bipolar Electrocoagulation - "COA-COMP", Neurosurg. Rev. (1984), pp. 187-190, describes a method for terminating electrosurgical power to the vessel so that charring of the vessel walls can be avoided.

By utilizing an electrosurgical forceps, a surgeon can either cauterize, coagulate/desiccate, reduce or slow bleeding and/or seal vessels by controlling the intensity, frequency and duration of the electrosurgical energy applied to the tissue. Generally, the electrical configuration of electrosurgical forceps can be categorized in two classifications: 1) monopolar electrosurgical forceps; and 2) bipolar electrosurgical forceps.

Monopolar forceps utilize one active electrode associated with the clamping end effector and a remote patient return electrode or pad which is typically attached externally to the patient. When the electrosurgical energy is applied, the energy travels from the active electrode, to the surgical site, through the patient and to the return electrode.

Bipolar electrosurgical forceps utilize two generally opposing electrodes which are disposed on the inner opposing surfaces of the end effectors and which are both electrically coupled to an electrosurgical generator. Each electrode is charged to a different electric potential. Since tissue is a conductor of electrical energy, when the effectors are utilized to grasp tissue therebetween, the electrical energy can be selectively transferred through the tissue.

In order to effect a proper seal with larger vessels, two predominant mechanical parameters must be accurately controlled - the pressure applied to the vessel and the gap between the electrodes both of which affect thickness of the sealed vessel. More particularly, accurate application of the pressure is important to oppose the walls of the vessel, to reduce the tissue impedance to a low enough value that allows enough electrosurgical energy through the tissue, to overcome the forces of expansion during tissue heating and to contribute to the end tissue thickness which is an indication of a good seal. It has been determined that a fused vessel wall is optimum between 0.001 and 0.006 inches. Below this range, the seal may shred or tear and above this range the lumens may not be properly or effectively sealed.

With respect to smaller vessel, the pressure applied to the tissue tends to become less relevant whereas the gap distance between the electrically conductive surfaces becomes more significant for effective sealing. In other words, the chances of the two electrically conductive surfaces touching during activation increases as the vessels become smaller.

Electrosurgical methods may be able to seal larger vessels using an appropriate electrosurgical power curve, coupled with an instrument capable of applying a large closure force to the vessel walls. It is thought that the process of coagulating small vessels is fundamentally different than electrosurgical vessel sealing. For the purposes herein, "coagulation" is defined as a process of desiccating tissue wherein the tissue cells are ruptured and dried and vessel sealing is defined as the process of liquefying the collagen in the tissue so that it reforms into a fused mass. Thus, coagulation of small vessels is sufficient to permanently close them. Larger vessels need to be sealed to assure permanent closure.

Numerous bipolar electrosurgical forceps have been proposed in the past for various open surgical procedures. However, some of these designs may not provide uniformly reproducible pressure to the blood vessel and may result in an ineffective or non-uniform seal. For example, U.S. Patent No. 2,176,479 to Willis, U.S. Patent Nos. 4,005,714 and 4,031,898 to Hiltebrandt, U.S. Patent Nos. 5,827,274, 5,290,287 and 5,312,433 to Boebel et al., U.S. Patent Nos. 4,370,980, 4,552,143, 5,026,370 and 5,116,332 to Lottick, U.S. Patent No. 5,443,463 to Stern et al., U.S. Patent No. 5,484,436 to Eggers et al. and U.S. Patent No. 5,951,549 to Richardson et al., all relate to electrosurgical instruments for coagulating, cutting and/or sealing vessels or tissue.

Many of these instruments include blade members or shearing members which simply cut tissue in a mechanical and/or electromechanical manner and are relatively ineffective for vessel sealing purposes. Other instruments rely on clamping pressure alone to procure proper sealing thickness and are not designed to take into account gap tolerances and/or parallelism and flatness requirements which are parameters which, if properly controlled, can assure a consistent and effective tissue seal. For example, it is known that it is difficult to adequately control thickness of the resulting sealed tissue by controlling clamping pressure alone for either of two reasons: 1) if too much force is applied, there is a possibility that the two poles will touch and energy will not be transferred through the tissue resulting in an ineffective seal; or 2) if too low a force is applied, a thicker less reliable seal is created.

A cordless electro-surgical forceps for sealing and/or cutting tissue is disclosed in US2009/240246 A1.

### SUMMARY

According to the invention an electrosurgical instrument according to claim 1 is provided. Preferred embodiments are defined in the dependent claims.

In the drawings and in the description that follows, the term "proximal", as is traditional, will refer to the end of electrosurgical instrument that is closer to the user, while the term "distal" will refer to the end that is further from the user.

### BRIEF DESCRIPTION OF THE DRAWINGS

Various embodiments of the subject instrument are described herein with reference to the drawings wherein:
Fig. 1 is a right, perspective view of a forceps;
Fig. 2 is an exploded view of the forceps of Fig. 1;
Fig. 3A is an exploded view of an end effector assembly of the forceps of Fig. 1;
Fig. 3B is a cross-sectional view of the end effector assembly of the forceps of Fig. 1;
Fig. 4A is a side view of the forceps of Fig. 1 with parts partially removed to show the electrical connection between a switch and the end effector assembly;
Fig. 4B is a left, perspective view of a jaw member of the end effector assembly of Fig. 1;
Fig. 4C is a left, perspective view of a jaw member of the end effector assembly of Fig. 1; and
Figs. 5A-5C are side views of the forceps of Fig. 1 illustrating actuation thereof between open and closed positions;
Fig. 6 is a side view of a knife for use with the forceps of Fig. 1 and
Figs. 7A and 7B are side views of forceps according to one embodiment of the present disclosure.

### DETAILED DESCRIPTION

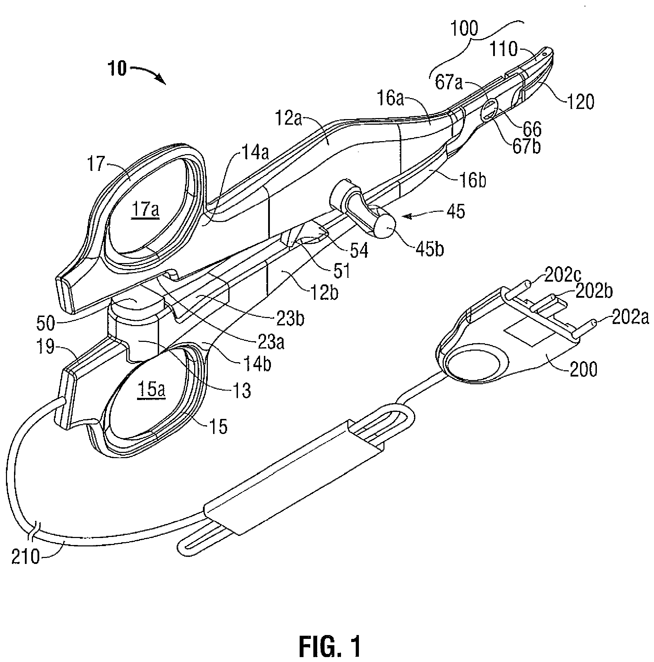

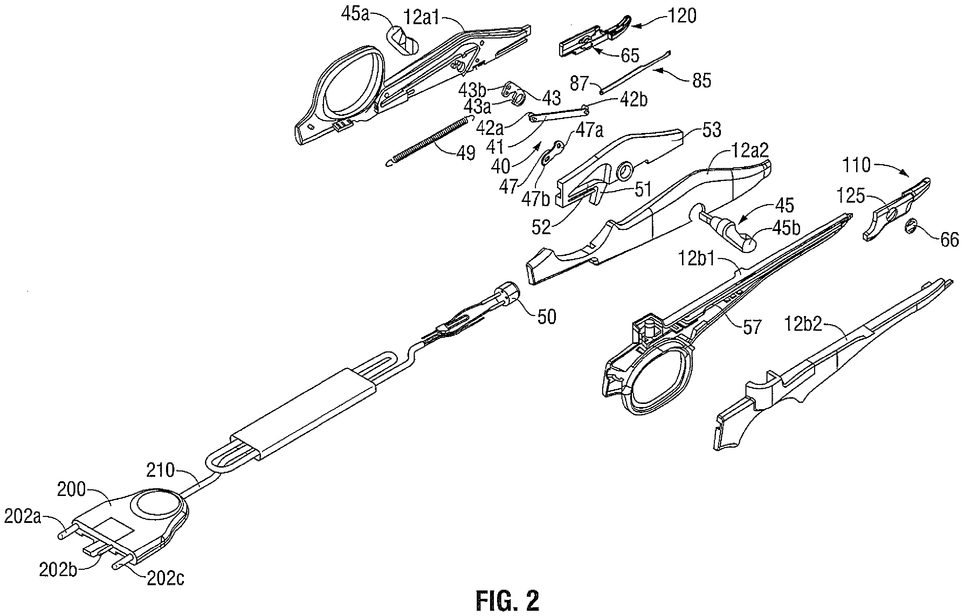

Referring initially to Figs. 1 and 2, a forceps 10 for use with open surgical procedures includes elongated shaft portions 12a and 12b each having a proximal end 14a, 14b and a distal end 16a and 16b, respectively.

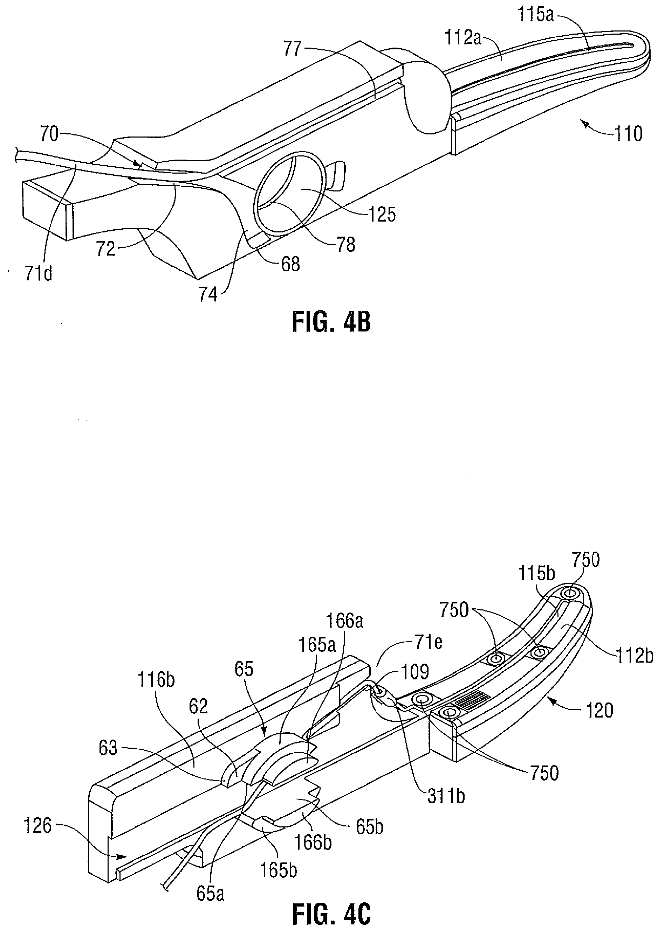

The forceps 10 includes an end effector assembly 100 that attaches to the distal ends 16a and 16b of shafts 12a and 12b, respectively. The end effector assembly 100 includes pair of opposing jaw members 110 and 120 that are pivotably connected and movable relative to one another about a pivot 65 (Fig. 2) to grasp tissue. Pivot 65 is disposed on a proximal end of jaw member 120 and includes opposing halves 65a and 65b disposed on opposing sides of a channel 126 (Fig. 4C) that is configured to facilitate reciprocation of a cutting mechanism or knife 85 therethrough (Fig. 2), as discussed in detail below.

Each shaft 12a and 12b includes a handle 15 and 17, respectively, disposed at the proximal end 14a and 14b thereof. Each handle 15 and 17 defines a finger hole 15a and 17a, respectively, therethrough for receiving a finger of the user. Handles 15 and 17 facilitate movement of the shafts 12a and 12b relative to one another which, in turn, pivot the jaw members 110 and 120 from an open position wherein the jaw members 110 and 120 are disposed in spaced relation relative to one another to a clamping or closed position wherein the jaw members 110 and 120 cooperate to grasp tissue therebetween.

As best seen in Fig. 2, shaft 12a is constructed from two components, namely, 12a1 and 12a2, that are coupled together to form shaft 12a. Likewise, shaft 12b is constructed from two components, namely, 12b1 and 12b2, that are coupled together to form shaft 12b. In some embodiments, component halves 12a1 and 12a2 and component halves 12b1 and 12b2 are ultrasonically welded together at a plurality of different weld points and/or may be mechanically coupled together by any suitable method including snap-fitting, adhesive, fastened, etc.

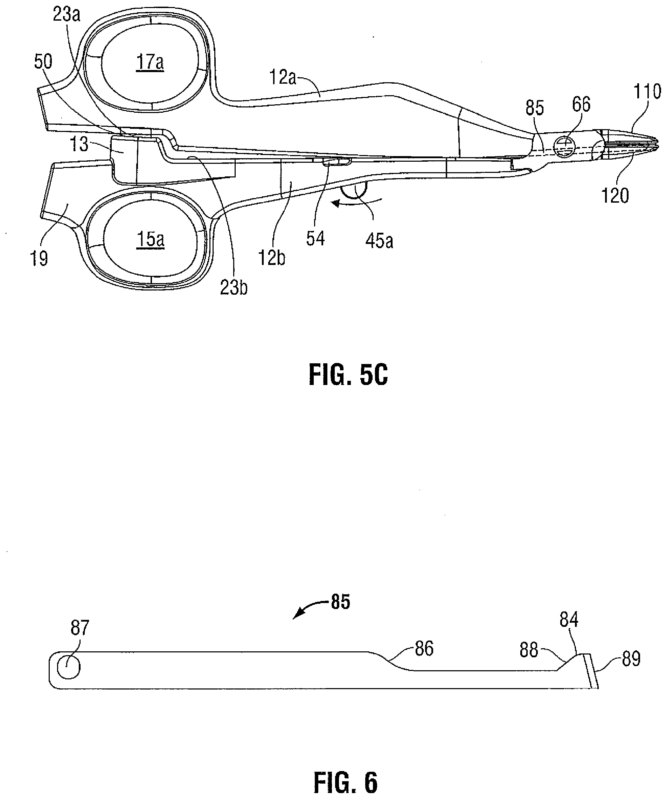

The arrangement of shaft 12b is slightly different from shaft 12a. More particularly, shaft 12a is generally hollow to house the knife 85 and an actuating mechanism 40. The actuating mechanism 40 is operatively associated with a trigger 45 having handle members 45a and 45b disposed on opposing sides of shaft 12a to facilitate left-handed and right-handed operation of trigger 45. Trigger 45 is operatively associated with a series of suitable inter-cooperating elements (e.g., Fig. 2 shows a trigger link 43, a knife pushing link 41, a spring 49, and an anti-deployment link 47) configured to mechanically cooperate (not explicitly shown) to actuate the knife 85 through tissue grasped between jaw members 110 and 120 upon actuation of trigger 45. The spring 49 is coupled between the inner structure of shaft 12b and the trigger link 43. Knife pushing link 41 includes a proximal pivot pin 42a that is received in a friction-fit manner within a pivot aperture 43b defined through the trigger link 43 and a distal pivot pin 42b that is received in a pivot aperture 87 (Fig. 6) defined through a proximal end of knife 85. The trigger 45 is operatively associated with the trigger link 43 such that rotation of the trigger 45 overcomes the biasing force of the spring 49 to rotate the trigger link 43 in a corresponding direction. The trigger link 43 mechanically cooperates with the knife pushing link 41 to reciprocate the knife 85 through knife channel 115 (Fig. 5C). Upon release of the trigger 45, the force of the spring 49 automatically rotates the trigger link 43 counter clock-wise to retract the knife 85 proximally.

The proximal end 14b of shaft 12b includes a switch cavity 13 protruding from an inner facing surface 23b of shaft 12b and configured to seat a depressible switch 50 therein (and the electrical components associated therewith). Switch 50 aligns with an opposing inner facing surface 23a of the proximal end 14a of shaft 12a such that upon approximation of shafts 12a and 12b toward one another, the switch 50 is depressed into biasing engagement with the opposing inner facing surface 23a of the proximal end 14a of shaft 12a.

As shown in Fig. 2, the actuating mechanism 40 includes a cover 53 disposed within the shaft 12a. The cover 53 includes a cantilever spring 52 extending distally from a proximal end thereof. A protrusion 51 is disposed on the distal end of cantilever spring 52 and extends from inner facing surface 23a of shaft 12a toward an opposing pad 54 disposed on inner facing surface 23b of shaft 12b (Fig. 1). Upon approximation of shafts 12a and 12b, protrusion 51 is engaged by pad 54 such that protrusion 51 is biased inward by virtue of the cantilever spring 52 toward shaft 12a to permit further approximation of shafts 12a and 12b and biasing engagement of switch 50 by the opposing inner facing surface 23a of the shaft 12a.

As shown in Fig. 1, an electrosurgical cable 210 having a plug 200 at its proximal end connects the forceps 10 to an electrosurgical generator (not shown). More specifically, the distal end of the cable 210 is securely held to the shaft 12b by a proximal shaft connector 19 and the proximal end of the cable 210 includes a plug 200 having prongs 202a, 202b, and 202c that are configured to electrically and mechanically engage the electrosurgical generator.

The tissue grasping portions of the jaw members 110 and 120 are generally symmetrical and include similar component features that cooperate to permit facile rotation about pivot 65 to effect the grasping and sealing of tissue. As a result, and unless otherwise noted, jaw member 110 and the operative features associated therewith are initially described herein in detail and the similar component features with respect to jaw member 120 will be briefly summarized thereafter.

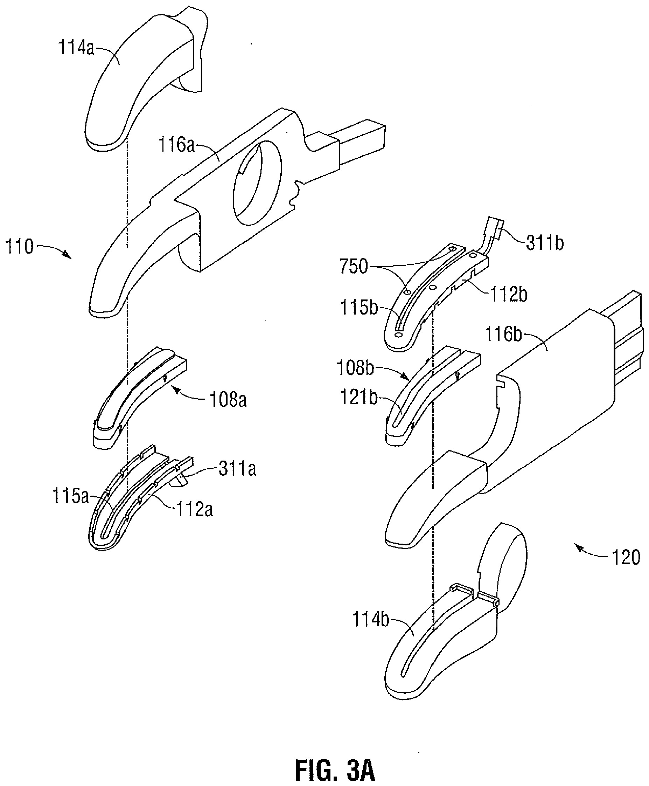

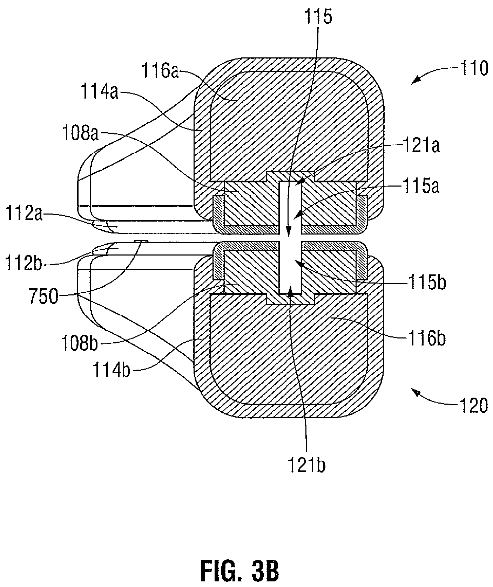

With reference to Figs. 3A and 3B, jaw member 110 includes an outer housing 116a, first and second non-conductive plastic insulators 108a and 114a, and an electrically conductive sealing surface 112a. The first and second insulators 108a and 114a are overmolded about jaw housing 116a in a two-shot overmolding process. More specifically, the first insulator 108a is overmolded about jaw housing 116a to electrically insulate the jaw housing 116a from sealing surface 112a and the second insulator 114a is overmolded about jaw housing 116a to secure the electrically conductive sealing surface 112a thereto. This may be accomplished by stamping, by overmolding, by overmolding a stamped sealing surface, and/or by overmolding a metal injection molded sealing surface. The jaw members 110 and 120 are made from a conductive material. In some embodiments, the jaw members 110 and 120 are powder coated with an insulative coating to reduce stray current concentrations during sealing.

As best shown by the cross-sectional view of Fig. 3B, electrically conductive sealing surface 112a of jaw member 110 is pronounced from the jaw housing 116a and the second insulator 114a such that tissue is grasped by the opposing electrically conductive sealing surfaces 112a and 112b when jaw members 110 and 120 are in the closed position.

Likewise, jaw member 120 includes similar elements that correspond to jaw member 110 including: an outer housing 116b, first and second plastic insulators 108b and 114b, and an electrically conductive sealing surface 112b that is pronounced from the jaw housing 116b and second insulator 114b. As described above with respect to jaw member 110, the first insulator 108b eleotrically insulates the jaw housing 116b from the sealing surface 112b and the second insulator 114b secures the sealing surface 112b to the jaw housing 116b. Insulators 114a and 114b extend along the entire length of jaw members 110 and 120, respectively, to reduce alternate or stray current paths during sealing. In some embodiments, each of sealing surfaces 112a and 112b may include an outer peripheral edge that has a radius such that each insulator 114a and 114b meets the respective sealing surface 112a and 112b along an adjoining edge that is generally tangential to the radius and/or meets along the radius.

As shown in Figs. 3A and 3B, at least one of the jaw members, e.g., jaw member 120, includes at least one stop member 750 disposed on the inner facing surfaces of the electrically conductive sealing surface 112b and/or 112a. Alternatively or in addition, the stop member(s) 750 may be disposed adjacent to the electrically conductive sealing surfaces 112a, 112b or proximate the pivot 65. The stop member(s) 750 facilitate gripping and manipulation of tissue and to define a gap between opposing jaw members 110 and 120 during sealing and cutting of tissue. In some embodiments, the stop member(s) 750 maintain a gap distance between opposing jaw members 110 and 120 within a range of about 0.001 inches (∼0.03 millimeters) to about 0.006 inches (∼0.015 millimeters).

As shown in Fig. 2, shaft 12b includes a beam 57 disposed therein and extending between handle 15 and jaw member 110. In some embodiments, the beam 57 is constructed of flexible steel to allow the user to generate additional sealing pressure on tissue grasped between the jaw members 110 and 120. More specifically, once end effector assembly 100 is closed about tissue, the shafts 12a and 12b may be squeezed toward each other to utilize the flexibility of the beam 57 to generate the necessary closure pressure between jaw members 110 and 120. In this scenario, the mechanical advantage realized by the compressive force associated with the beam 57 facilitates and assures consistent, uniform, and accurate closure pressure about tissue grasped between jaw members 110 and 120 (e.g., within a working pressure range of about 3 kg/cm² to about 16 kg/cm²). By controlling the intensity, frequency, and duration of the electrosurgical energy applied to the tissue, the user can seal tissue. In some embodiments, the gap distance between opposing sealing surfaces 112a and 112b during sealing ranges from about 0.001 inches to about 0.005 inches.

The sealing surfaces 112a and 112b are relatively flat to avoid current concentrations at sharp edges and to avoid arcing between high points. In addition, and due to the reaction force of the tissue when engaged, each of jaw members 110 and 120 may be manufactured to resist bending, e.g., tapered along its length to provide a constant pressure for a constant tissue thickness at parallel and the thicker proximal portion of the jaw members 110 and 120 will resist bending due to the reaction force of the tissue.

As shown in Figs. 3A, 3B, 4B, and 4C, at least one of jaw members 110 and 120 includes a knife channel 115a and/or 115b, respectively, disposed therebetween that is configured to allow reciprocation of a knife 85 therethrough. In the illustrated embodiment, a complete knife channel 115 is formed when two opposing channel halves 115a and 115b associated with respective jaw members 110 and 120 come together upon grasping of the tissue. Each plastic insulator 108a and 108b includes a trough 121a and 121b, respectively, that aligns in vertical registration with an opposing knife channel half 115a and 115b, respectively, such that knife 85 does not contact or cut through plastic insulators 108a and 108b upon reciprocation through knife channel 115. The width of knife channels 115a and 115b and their respective troughs 121a and 121b may be equal along an entire length thereof.

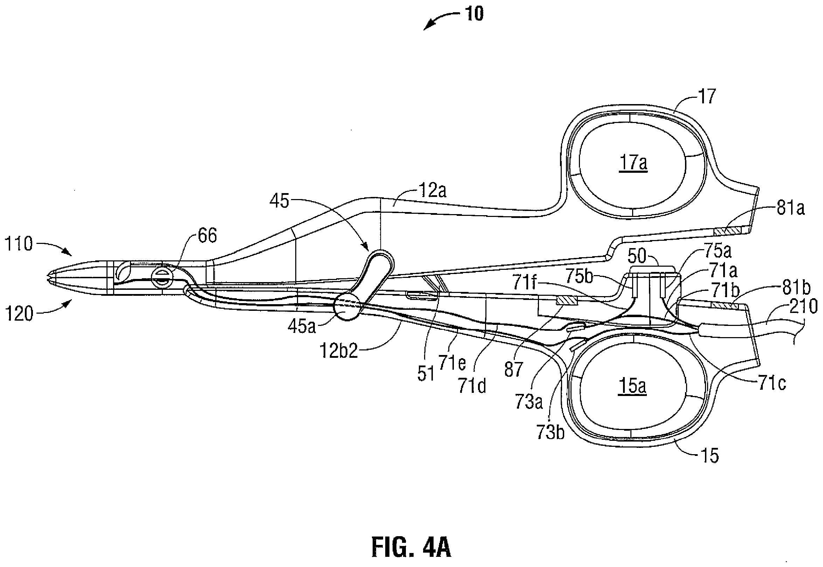

As best shown in Fig. 4A, the interior of cable 210 houses leads 71a, 71b and 71c. Leads 71a, 71b, and 71c extend from the plug 200 through cable 210 and exit the distal end of the cable 210 within the proximal connector 19 of shaft 12b. More specifically, lead 71a is interconnected between prong 202b and a first terminal 75a of the switch 50. Lead 71b is interconnected between prong 202c and a solder sleeve 73a which, in turn, connects lead 71b to an RF lead 71d and to a second terminal 75b of the switch 50 via a connector lead 71f. RF lead 71d carries a first electrical potential of electrosurgical energy from lead 71b to sealing surface 112a. Lead 71c is interconnected between prong 202a and a solder sleeve 73b which, in turn, connects lead 71c to an RF lead 71e. RF lead 71e carries a second electrical potential of electrosurgical energy from lead 71c to sealing surface 112b,

With reference to Fig. 4B, a lead channel 77 is defined in the proximal end of jaw member 110 to provide a pathway for lead 71d to connect to a junction 311a (Fig. 3A) extending from a proximal end of sealing surface 112a. A proximal end of lead channel 77 opens into a raceway 70 that includes a generally elongated configuration with a narrowed proximal end 72 and a broadened distal end 74 that defines an arcuate sidewall 68. Lead 71d is routed to follow a path through the proximal end 72 of raceway 70 and, further, through lead channel 77 for connection to junction 311a.

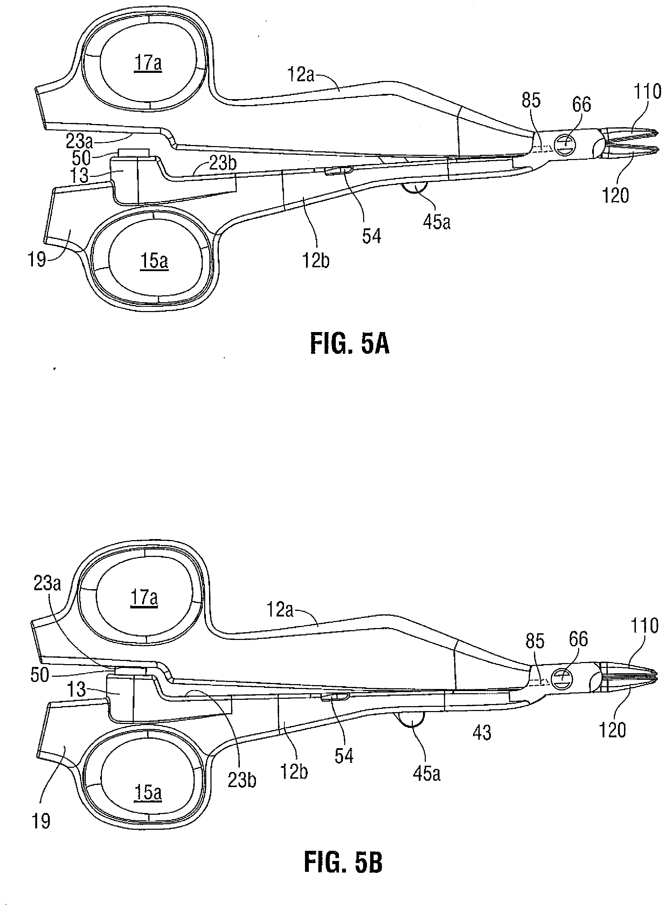

With reference to Fig. 4C, pivot halves 65a and 65b are disposed on opposing sides of channel 126 to facilitate translation of the knife 85 therethrough (Figs. 5A-5C). Pivot halves 65a and 65b are disposed in a split spherical configuration and each include a respective base portion 165a and 165b that support an extension portion 166a and 166b thereon, respectively. Extension portions 166a and 166b are configured to engage correspondingly-dimensioned apertures 67a and 67b, respectively, disposed through pivot plate 66 to pivotably secure jaw member 110 to jaw member 120. A lead channel 109 is defined in the proximal end of jaw member 120 to provide a pathway for lead 71e to connect to a junction 311b extending from a proximal end of sealing surface 112b. Lead 71e is routed to follow a path through raceway 70 and, further between opposing pivot halves 65a and 65b and through lead channel 109 for connection to junction 311b.

With reference to Figs. 5A-5C, as the user applies closure pressure on shafts 12a and 12b to depress switch 50 (Fig. 5B), a first threshold is met corresponding to the closure force applied to switch 50 as a function of displacement of switch 50 that causes switch 50 to generate a first tactile response that corresponds to a complete grasping of tissue disposed between jaw members 110 and 120. Following the first tactile response, as the user applies additional closure pressure on shafts 12a and 12b (Fig. 5C), a second threshold is met corresponding the closure force applied to switch 50 as a function of displacement of switch 50 that causes the switch 50 to generate a second tactile response that corresponds to a signal being generated to the electrosurgical generator to supply electrosurgical energy to the sealing surfaces 112a and 112b. More specifically, the second tactile response indicates closing of a normally open circuit between switch terminals 75a and 75b and, in turn, establishment of an electrical connection between leads 71a and 71b. As a result of the electrical connection between leads 71a and 71b, the electrosurgical generator senses a voltage drop between prongs 202b and 202c and, in response thereto, supplies electrosurgical energy to sealing surfaces 112a and 112b via leads 71d and 71e, respectively.

The first tactile response indicates to the user that the maximum grasping pressure has been reached before end effector 100 is energized where the user is free to approximate, manipulate, and grasp tissue as needed. In this scenario, the second tactile response indicates to the user the electrosurgical activation of the end effector 100. The switch 50 may include a plurality of other tactile responses between the above discussed first and second tactile responses and/or subsequent to the second tactile response that correspond to particular functions of the forceps 10 such as, for example, operation of the knife 85 and/or the actuation assembly 40, operation of a safety lockout mechanism associated with the actuation assembly 40, as discussed in detail below.

As shown in Fig. 4A, forceps 10 may include a gauge or sensor element 87 disposed within one or both of shafts 12a, 12b such that the clamping or grasping forces being applied to target tissue by end effector 100 may be measured and/or detected. For example, in some embodiments, sensor element 87 may be a strain gauge 87 operably associated with one or both jaw members 110, 120. Sensor element 87 may be one or more Hall effect sensors or strain gauges such as, for example, metallic strain gauges, piezoresistive strain gauges, that may be disposed within one or both of shafts 12a and 12b and/or within one or both of jaw members 110 and 120 to detect tissue pressure. Metallic strain gauges operate on the principle that as the geometry (e.g., length, width, thickness, etc.) of the conductive material changes due to mechanical stress, the resistance of the conductive material changes as a function thereof. This change in resistance is utilized to detect strain or applied mechanical stress such as, for example, the mechanical stress applied to tissue by jaw members 110 and 120. Piezoresistive strain gauges operate based on the changing resistivity of a semiconductor due to the application of mechanical stress.

Hall effect sensors may be incorporated to determine the gap between jaw members 110 and 120 based on a detected relationship between the magnetic field strength between jaw members 110 and 120 and the distance between jaw members 110 and 120.

One or more reed switches 81a, 81b may be incorporated within shafts 12a and 12b to determine the proximity thereof relative to one another, as shown in Fig, 4A. More specifically, the reed switch(s) may be comprised of a switch 81a disposed within one of the shafts (e.g., shaft 12a) and a magnetic element 81b (e.g., electromagnet, permanent magnet, coil, etc.) disposed within the opposing shaft (e.g., shaft 12a) such that upon approximation of shafts 12a and 12b, the reed switch 81a is activated or closed by the magnetic field of the magnetic element 81b and, likewise, as shafts 12a and 12b are moved away from each other, the lack of magnetic field operates to deactivate or open the reed switch 81a. In this manner, the proximity of shafts 12a and 12b and thus, jaw members 110 and 120, may be determined based on the reaction of the reed switch 81a to the magnetic element 81b.

Any of the above discussed sensors, switches, and/or strain gauge(s) may be incorporated within an electrical circuit such that the strain detected by the strain gauge changes the electrical signal through the circuit. With this purpose in mind, an electrical circuit between the strain gauge and the switch 50 and/or an electrosurgical generator (not shown) allows communication of information such as desired tissue pressure thereto. This information may be tied to the activation of switch 50 such that the switch is not activated until a desired and/or predetermined pressure on tissue grasped between jaw members 110 and 120 is achieved as detected by the strain gauge. Accordingly, the strain gauge may be disposed strategically on the forceps 10, e.g., on one or more of jaw members 110, 120, such that pressure applied to tissue grasped between jaw members 110 and 120 affects the strain gauge.

In use, forceps 10 may be calibrated such that particular tactile responses (e.g., the first tactile response) of switch 50 corresponds to a predetermined grasping pressure on tissue as determined through use of one or more of the above discussed sensors, switches, and/or strain gauge(s). The predetermined grasping pressure about tissue is within the range of about 3 kg/cm² to about 16 kg/cm² in one embodiment and, in another embodiment, about 7 kg/cm² to about 13 kg/cm². In some embodiments, switch 50 may generate multiple tactile responses, each of which corresponds to different predetermined grasping force. For a more detailed discussion of force sensing and/or measuring devices such as load cells, strain gauges, etc., reference is made to commonly-owned U.S. Application No. 11/409,154, filed on April 21, 2006.

As shown in Figs. 2, 4B, and 4C, the pivot 65 connects through an aperture 125 defined through jaw member 120 and matingly engages a pivot plate 66 seated within a circumferential lip or flange 78 (Fig. 4B) defined around the periphery of aperture 125 such that the pivot 65 is rotatably movable within the aperture 125 to move jaw members 110 and 120 between open and closed positions.

In some embodiments, actuation of the knife 85 is associated with activation of the switch 50. For example, sensor 87 may be embodied as a position sensor configured to detect the position of knife 85 relative to jaw members 110 and 120 and/or relative to tissue held therebetween. Additionally or alternatively, sensor 87 may be configured to detect either of the first and second tactile responses of switch 50 and allow or prevent actuation of the knife 85 accordingly. For example, based on feedback from the sensor 87, any one or more inter-cooperating elements or lockout mechanisms associated with the actuating mechanism 40 may be energized or deenergized to allow or prevent actuation of the knife 85, as described in more detail below.

As shown in Fig. 6, knife 85 includes a step 86 that reduces the profile of the knife 85 toward a distal end thereof. The distal end of the knife 85 has a step 88 that increases the profile of the knife 85 toward a sharpened distal cutting edge 89. The knife 85 includes a chamfered portion 84 where the sharpened distal cutting edge 89 meets the step 88 to facilitate smooth retraction of knife 85 through the knife channel 15.

The forceps 10 may include a safety lockout mechanism having a series of suitable inter-cooperating elements (e.g., anti-deployment link 47, trigger link 43) that work together to prevent unintentional firing of the knife 85 when the jaw members 110 and 120 are disposed in the open position. Generally, the anti-deployment link 47 mechanically cooperates with the trigger link 43 to prevent advancement of the knife 85 until the jaw members 110 and 120 are closed about tissue. More specifically, the anti-deployment link 47 includes an engagement tooth 47a formed thereon that is releasably received within a notch portion 43a formed in the trigger link 43 (Fig. 2) when the jaw members 110 and 120 are in the open position such that clock-wise rotation of trigger link 43 is prevented so that the knife 85 can not be advanced distally when the jaw members 110 and 120 are in the open position (Fig. 5A). When the jaw members 110 and 120 are moved to the closed position as illustrated in Fig. 5B, the safety lockout mechanism automatically disengages to allow selective actuation of the knife 85. More particularly, a pin (not shown) disposed on protrusion 51 is received within an aperture 47b defined through anti-deployment link 47 to couple anti-deployment link 47 to protrusion 51. In this way, upon closure of jaw members 110 and 120, the protrusion 51 contacts the pad 54 and is biased inward toward shaft 12b, thereby causing counter clock-wise rotation of protrusion 51 and, in turn, clock-wise rotation of the anti-deployment link 47 such that the engagement tooth 47a is released from engagement with the notch portion 43a. Disengagement of tooth 47a from notch portion 43a allows actuation of the trigger 45 to advance the knife 85 distally through knife channel 15 to cut tissue grasped between jaw members 110 and 120, An example of a safety lockout mechanism for use with forceps 10 is described in commonly-owned U.S. Application Serial No, 12/896,100 entitled "Blade Deployment Mechanisms for Surgical Forceps", filed on October 1, 2010.

Any one or more of the inter-cooperating elements of the safety lockout mechanism (e.g., anti-deployment link 47) may be electrically interconnected to the switch 50 and include suitable electro-mechanical components (e.g., springs, rods, solenoids, etc.) configured to be energized via activation of the switch 50 (e.g., via any one of leads 71a, 71b, 71c, 71d, 71e) to mechanically manipulate the safety lockout mechanism. For example, upon electrical conduction through leads 71d and 71e to energize the end effector 100, the anti-deployment link 47 is energized to cause actuation thereof such that the safety lockout mechanism disengages to allow selective actuation of the knife 85. In this scenario, by way of example, selective actuation of the knife 85 may be prevented until switch 50 has been depressed to generate at least the first tactile response.

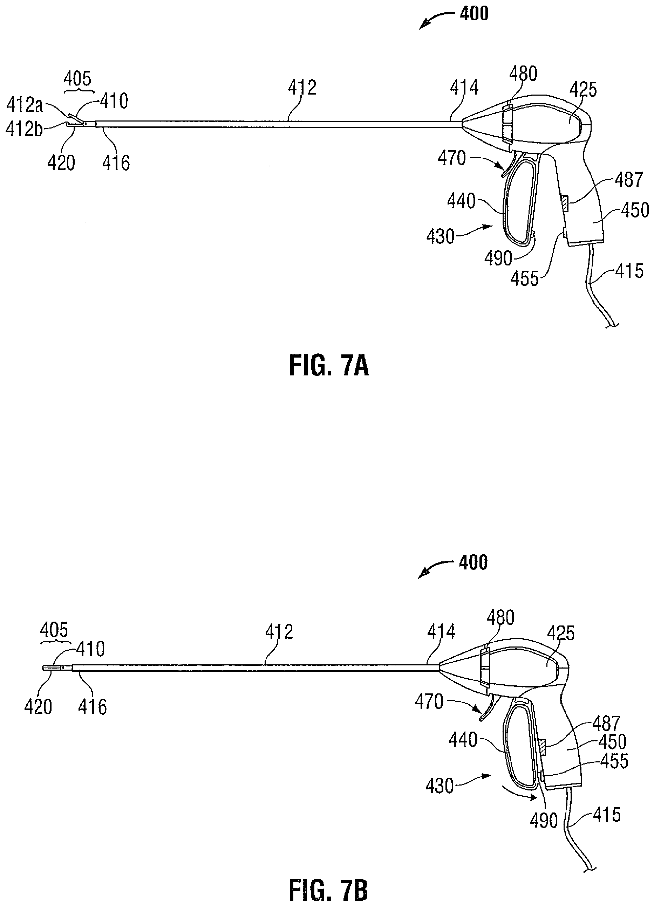

Referring now to Figs. 7A and 7B, a forceps 400 for use with endoscopic surgical procedures is shown in accordance with embodiments of the present disclosure. Forceps 400 operates substantially as described above with respect to forceps 10 (see Figs. 1-6) and will only be discussed to the extent necessary to describe the differences between the embodiments.

Generally, forceps 400 includes a housing 425, a handle assembly 430, a rotating assembly 480, and an end effector assembly 405 that mutually cooperate to grasp, seal, and divide tubular vessels and vascular tissue. The forceps 400 includes a shaft 412 that has a distal end 416 configured to mechanically engage the end effector assembly 405 and a proximal end 414 that mechanically engages the housing 425.

The handle assembly 430 includes a fixed handle 450 and a movable handle 440. Fixed handle 450 is integrally associated with housing 425 and handle 440 is movable relative to fixed handle 450. Rotating assembly 480 is associated with the housing 425 and is rotatable in either direction about a longitudinal axis "A-A". The housing 425 houses the internal working components of the forceps 400.

End effector assembly 405 is coupled to the distal end 416 of shaft 412 and includes a pair of opposing jaw members 410 and 420. Jaw members 410 and 420 each include an electrically conductive tissue sealing surface 412a and 412b, respectively, disposed thereon. Movable handle 440 of handle assembly 430 is operably associated with the end effector assembly 405 through a suitable drive assembly (not shown) to impart movement of the jaw members 410 and 420 from an open position to a clamped or closed position. The drive assembly may be any suitable combination of mechanical, electrical, and/or electromechanical components disposed within the housing 425 and configured to operably associate the movable handle 440 with the end effector assembly 405 such that movement of the movable handle 440 relative to the fixed handle 450 effects movement of the jaw members 410 and 420.

As substantially described above with respect to the jaw members 110 and 120, at least one of the jaw members 410 and 420 includes at least one stop member (not explicitly shown) disposed on the inner facing surfaces of the electrically conductive sealing surface 412b and/or 412a to facilitate gripping and manipulation of tissue and to define a gap between the jaw members 410 and 420 during sealing and cutting of tissue. For example, the stop member(s) may maintain a gap distance between the jaw members 410 and 420 of about 0.001 inches (∼0,03 millimeters) to about 0.006 inches (∼0.015 millimeters).

An electrosurgical cable 415 is coupled at a proximal end to the fixed handle 450 and connects the forceps 400 to an electrosurgical generator (not shown). Electrosurgical cable 415 houses a plurality of electrical leads (e.g., leads 71a, 71b and 71c) that cooperatively operate to supply electrosurgical energy to the electrically conductive sealing surfaces 412a and 412b as substantially described hereinabove with respect to electrosurgical cable 210 of Fig. 1.

Forceps 400 includes a switch 455 disposed on the fixed handle 450 that operates substantially as described above with respect to switch 50 of forceps 10 (see Figs. 1-5C). Although depicted on a lower end of the fixed handle 450 in Figs. 7A and 7B, switch 455 or a similar type switch may be disposed on any suitable location of the fixed handle 450 and, thus, Figs. 7A and 7B are not intended to be limiting with respect to the location of switch 455 on the fixed handle 450.

The lower end of the movable handle 440 includes a switch engaging surface 490 extending therefrom. The switch engaging surface 490 is disposed on a surface of the movable handle 440 that faces the fixed handle 450. When the movable handle 440 is moved toward the fixed handle 450, the switch engaging surface 490 substantially aligns with the switch 455 such that as the moveable handle 440 is moved or squeezed toward the fixed handle 450, as indicated by the rotational arrow depicted in Fig. 7B, the switch 455 is depressed via biasing engagement with the switch engaging surface 490. As the user applies closure pressure on the moveable handle 440 to depress the switch 455 (not explicitly shown), a first threshold is met corresponding to the closure force applied to the switch 455 as a function of displacement of the switch 455 that causes the switch 455 to generate a first tactile response that corresponds to a complete grasping of tissue disposed between jaw members 410 and 420. Following the first tactile response, as the user applies additional closure pressure on the moveable handle 440 (not explicitly shown), a second threshold is met corresponding the closure force applied to the switch 455 as a function of displacement of the switch 455 that causes the switch 455 to generate a second tactile response that corresponds to a signal being generated to the electrosurgical generator to supply electrosurgical energy to the jaw members 410 and 420.

Engagement of the switch 455 by switch engaging surface 490 depresses the switch 455 to meet any one or more thresholds as a function of displacement of switch 455 that, as described above with reference to switch 50 of Fig. 1, causes the switch 455 to generate one or more tactile responses. Each tactile response corresponds to a particular condition. For example, the switch 455 may generate a first tactile response corresponding to a complete grasping of tissue sensed between jaw members 410 and 420 and a second tactile response upon additional depression of the switch 455 relative to the fixed handle 450 corresponding to a signal being generated to the electrosurgical generator to supply electrosurgical energy to the sealing surfaces 412a and 412b.

Although not explicitly shown, at least one of the jaw members 410 and 420 may include a knife channel that is configured to allow reciprocation of a knife therethrough as substantially described above with respect to the knife channel 115a and/or 115b and the knife 85 of Figs. 1-6. Forceps 400 includes a trigger 470 operatively associated with a series of suitable inter-cooperating elements (not explicitly shown) configured to actuate the knife substantially as described above with respect to trigger 45 of Figs. 1-6.

Substantially as described above with respect to the safety lockout mechanism of the forceps 10, forceps 400 may include a safety lockout mechanism having a series of suitable inter-cooperating elements that work together to prevent unintentional firing of the knife when the jaw members 410 and 420 are disposed in the open position. By way of example, the forceps 400 may include inter-cooperating elements such as or substantially similar to the anti-deployment link 47 and trigger link 43 described hereinabove to prevent advancement of the knife until the jaw members 410 and 420 are closed about tissue as substantially described above with respect to the safety lockout mechanism of the forceps 10. An example of a safety lockout mechanism for use with forceps 400 is described in commonly-owned U.S. Application Serial No. 12/896,100 entitled "Blade Deployment Mechanisms for Surgical Forceps," filed on October 1, 2010.

In some embodiments, any one or more of the inter-cooperating elements of the safety lockout mechanism may be electrically interconnected to the switch 455 and include suitable electro-mechanical components (e.g., springs, rods, solenoids, etc.) configured to be energized via activation of the switch 455 to mechanically manipulate the safety lockout mechanism as substantially described above with respect to the switch 50 of the forceps 10.

Substantially as described above with respect to the sensor element 87 of Fig. 4A, forceps 400 may optionally include a gauge or sensor element 487 disposed within either movable handle 440 or fixed handle 450 (depicted within fixed handle 450 in Figs. 7A and 7B for illustrative purposes) such that the clamping or grasping forces being applied to target tissue by end effector 405 may be measured. For example, in some embodiments, the sensor element 487may be a strain gauge operably associated with one or both jaw members 410, 420.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An electrosurgical instrument (400), comprising:
a housing (425);
an elongated shaft (412) extending from the housing and having a pair of opposing jaw members (410, 420) disposed at a distal end (416) thereof, each of the jaw members including an electrically conductive sealing surface (412a, 412b) configured to deliver energy to tissue;
a trigger (470) operably coupled to the housing;
a knife (85) operably coupled to the trigger such that actuation of the trigger translates the knife distally through a knife channel extending through at least one of the jaw members;
a handle assembly (430) operably coupled to the housing and including a movable handle (440) configured to move relative to the housing to effect movement of the jaw members between an open position and a closed position; and **characterised by**
a switch (455) disposed on the housing and configured to be depressed by the movable handle when the jaw members are in the closed position to control a supply of electrosurgical energy from an electrosurgical energy source to each of the electrically conductive sealing surfaceswherein the switch is configured to be depressed to a first threshold corresponding to a grasping of tissue and to be further depressed to a second threshold to activate a supply of electrosurgical energy to each of the electrically conductive sealing surfaceswherein the switch is configured to generate a first tactile response when the switch is depressed to the first threshold and a second tactile response when the switch is depressed to the second threshold.

2. The electrosurgical instrument according to claim 1, further comprising a stop member disposed on the electrically conductive sealing surface of at least one of the jaw members, the stop member configured to maintain a distance between the electrically conductive sealing surfaces when the jaw members are in the closed position.

3. The electrosurgical instrument according to claim 1, further comprising a safety lockout configured to prevent distal translation of the knife (85) when the jaw members are disposed in the open position.

4. The electrosurgical instrument according to claim 3, wherein the safety lockout mechanism includes an anti-deployment link (47) configured to cooperate with a trigger link (43) to prevent distal translation of the knife (85) when the jaw members are disposed in the open position.

5. The electrosurgical instrument according to claim 4, wherein the anti-deployment link (47) includes an engagement tooth (47a) configured to engage a notch portion (43a) formed in the trigger link (43) when the jaw members are in the open position to prevent rotation of the trigger link (43) and distal translation of the knife (85).

6. The electrosurgical instrument according to claim 5, wherein the engagement tooth is configured to disengage from the notch portion formed in the trigger link upon movement of the jaw members to the closed position to allow rotation of the trigger link (43) and distal translation of the knife (85).

7. The electrosurgical instrument according to claim 1, further comprising a switch engaging surface (490) disposed on the movable handle and configured to engage the switch upon movement of the jaw members to the closed position.

8. The electrosurgical instrument according to claim 1, further comprising a sensor element (487) disposed on the housing and configured to measure a grasping force applied on tissue grasped between the jaw members when the jaw members are in the closed position.

## Patentansprüche

1. Elektrochirurgisches Instrument (400), das Folgendes umfasst:
ein Gehäuse (425);
einen länglichen Schaft (412), der sich von dem Gehäuse aus erstreckt und ein Paar gegenüberliegender Backenelemente (410, 420) aufweist, die an einem distalen Ende (416) davon angeordnet sind, wobei jedes der Backenelemente eine elektrisch leitfähige Dichtungsoberfläche (412a, 412b) beinhaltet, die konfiguriert ist, um Energie an Gewebe abzugeben;
einen Auslöser (470), der mit dem Gehäuse wirkgekoppelt ist;
ein Messer (85), das derart mit dem Auslöser wirkgekoppelt ist, dass eine Betätigung des Auslösers das Messer durch einen Messerkanal distal verschiebt, der sich durch wenigstens eines der Backenelemente erstreckt;
eine Griffanordnung (430), die mit dem Gehäuse wirkgekoppelt ist und einen bewegbaren Griff (440) beinhaltet, der konfiguriert ist, um sich relativ zu dem Gehäuse zu bewegen, um eine Bewegung der Backenelemente zwischen einer geöffneten Position und einer geschlossenen Position zu bewirken; und **gekennzeichnet durch**
einen Schalter (455), der an dem Gehäuse angeordnet und konfiguriert ist, um durch den bewegbaren Griff niedergedrückt zu werden, wenn sich die Backenelemente in der geschlossenen Position befinden, um eine Zufuhr von elektrochirurgischer Energie von einer elektrochirurgischen Energiequelle zu jeder der elektrisch leitfähigen Dichtungsoberflächen zu steuern, wobei der Schalter konfiguriert ist, um auf einen ersten Schwellenwert niedergedrückt zu werden, der einem Greifen von Gewebe entspricht, und um ferner auf einen zweiten Schwellenwert niedergedrückt zu werden, um eine Zufuhr von elektrochirurgischer Energie zu jeder der elektrisch leitfähigen Dichtungsoberflächen zu aktivieren, wobei der Schalter konfiguriert ist, um eine erste taktile Reaktion, wenn der Schalter auf den ersten Schwellenwert niedergedrückt wird, und eine zweite taktile Reaktion zu erzeugen, wenn der Schalter auf den zweiten Schwellenwert niedergedrückt wird.

2. Elektrochirurgisches Instrument nach Anspruch 1, das ferner ein Anschlagelement umfasst, das auf der elektrisch leitfähigen Dichtungsoberfläche von wenigstens einem der Backenelemente angeordnet ist, wobei das Anschlagelement konfiguriert ist, um einen Abstand zwischen den elektrisch leitfähigen Dichtungsoberflächen aufrechtzuerhalten, wenn sich die Backenelemente in der geschlossenen Position befinden.

3. Elektrochirurgisches Instrument nach Anspruch 1, das ferner eine Sicherheitssperre umfasst, die konfiguriert ist, um eine distale Verschiebung des Messers (85) zu verhindern, wenn die Backenelemente in der geöffneten Position angeordnet sind.

4. Elektrochirurgisches Instrument nach Anspruch 3, wobei der Sicherheitssperrmechanismus ein Anti-Entfaltungsverbindungsglied (47) beinhaltet, das konfiguriert ist, um mit einem Auslöserverbindungsglied (43) zusammenzuwirken, um die distale Verschiebung des Messers (85) zu verhindern, wenn die Backenelemente in der geöffneten Position angeordnet sind.

5. Elektrochirurgisches Instrument nach Anspruch 4, wobei das Anti-Entfaltungsverbindungsglied (47) einen Eingriffszahn (47a) beinhaltet, der konfiguriert ist, um einen Kerbabschnitt (43a) in Eingriff zu nehmen, der in dem Auslöserverbindungsglied (43) ausgebildet ist, wenn sich die Backenelemente in der geöffneten Position befinden, um eine Drehung des Auslöserverbindungsglieds (43) und die distale Verschiebung des Messers (85) zu verhindern.

6. Elektrochirurgisches Instrument nach Anspruch 5, wobei der Eingriffszahn konfiguriert ist, um sich von dem Kerbabschnitt, der in dem Auslöserverbindungsglied ausgebildet ist, bei der Bewegung der Backenelemente in die geschlossene Position zu lösen, um die Drehung des Auslöserverbindungsglieds (43) und die distale Verschiebung des Messers (85) zu ermöglichen.

7. Elektrochirurgisches Instrument nach Anspruch 1, das ferner eine Schaltereingriffsoberfläche (490) umfasst, die an dem bewegbaren Griff angeordnet und konfiguriert ist, um den Schalter bei der Bewegung der Backenelemente in die geschlossene Position in Eingriff zu nehmen.

8. Elektrochirurgisches Instrument nach Anspruch 1, das ferner ein Sensorelement (487) umfasst, das an dem Gehäuse angeordnet und konfiguriert ist, um eine Greifkraft zu messen, die an Gewebe angewendet wird, das zwischen den Backenelementen gegriffen wird, wenn sich die Backenelemente in der geschlossenen Position befinden.

## Revendications

1. Instrument électrochirurgical (400) comprenant :
un boîtier (425) ;
un arbre allongé (412) s'étendant depuis le boîtier et ayant une paire d'éléments de mâchoire opposés (410, 420) disposés à une extrémité distale (416) de celui-ci, chacun des éléments de mâchoire comportant une surface d'étanchéité électriquement conductrice (412a, 412b) configurée pour fournir de l'énergie aux tissus ;
une gâchette (470) accouplée de manière fonctionnelle au boîtier ;
un couteau (85) accouplé de manière fonctionnelle à la gâchette de telle sorte que l'actionnement de la gâchette déplace le couteau de manière distale à travers un canal de couteau s'étendant à travers au moins l'un des éléments de mâchoire ;
un ensemble poignée (430) accouplé de manière fonctionnelle au boîtier et comportant une poignée mobile (440) conçue pour se déplacer par rapport au boîtier afin d'effectuer le mouvement des éléments de mâchoire entre une position ouverte et une position fermée ; et **caractérisé par**
un interrupteur (455) disposé sur le boîtier et conçu pour être enfoncé par la poignée mobile lorsque les éléments de mâchoire sont en position fermée afin de commander une alimentation en énergie électrochirurgicale à partir d'une source d'énergie électrochirurgicale vers chacune des surfaces d'étanchéité électriquement conductrices, l'interrupteur étant conçu pour être enfoncé jusqu'à un premier seuil correspondant à une saisie de tissu et pour être en outre enfoncé jusqu'à un second seuil pour activer une alimentation en énergie électrochirurgicale à chacune des surfaces d'étanchéité électriquement conductrices, l'interrupteur étant conçu pour générer une première réponse tactile lorsque l'interrupteur est enfoncé jusqu'au premier seuil et une seconde réponse tactile lorsque l'interrupteur est enfoncé jusqu'au second seuil.

2. Instrument électrochirurgical selon la revendication 1, comprenant en outre un élément d'arrêt disposé sur la surface d'étanchéité électriquement conductrice d'au moins l'un des éléments de mâchoire, l'élément d'arrêt étant conçu pour maintenir une distance entre les surfaces d'étanchéité électriquement conductrices lorsque les éléments de mâchoire se trouvent dans la position fermée.

3. Instrument électrochirurgical selon la revendication 1, comprenant en outre un verrouillage de sécurité conçu pour empêcher la translation distale du couteau (85) lorsque les éléments de mâchoire sont disposés dans la position ouverte.

4. Instrument électrochirurgical selon la revendication 3, dans lequel le mécanisme de verrouillage de sécurité comporte une liaison anti-déploiement (47) conçue pour coopérer avec une liaison de déclenchement (43) afin d'empêcher la translation distale du couteau (85) lorsque les éléments de mâchoire sont disposés dans la position ouverte.

5. Instrument électrochirurgical selon la revendication 4, dans lequel la liaison anti-déploiement (47) comporte une dent de mise en prise (47a) conçue pour se mettre en prise avec une partie d'encoche (43a) formée dans la liaison de déclenchement (43) lorsque les éléments de mâchoire sont dans la position ouverte afin d'empêcher la rotation de la liaison de déclenchement (43) et la translation distale du couteau (85).

6. Instrument électrochirurgical selon la revendication 5, dans lequel la dent de mise en prise est conçue pour ne plus être en prise avec la partie d'encoche formée dans la liaison de déclenchement lors du déplacement des éléments de mâchoire vers la position fermée afin de permettre la rotation de la liaison de déclenchement (43) et la translation distale du couteau (85).

7. Instrument électrochirurgical selon la revendication 1, comprenant en outre une surface de mise en prise de l'interrupteur (490) disposée sur la poignée mobile et conçue pour se mettre en prise avec l'interrupteur lors du déplacement des éléments de mâchoire vers la position fermée.

8. Instrument électrochirurgical selon la revendication 1, comprenant en outre un élément capteur (487) disposé sur le boîtier et conçu pour mesurer une force de préhension appliquée sur du tissu saisi entre les éléments de mâchoire lorsque les éléments de mâchoire sont en position fermée.
